# EUROPÄISCHE PATENTSCHRIFT

(11) **EP 2 079 680 B1**
(45) Veröffentlichungstag und Bekanntmachung des Hinweises auf die Patenterteilung: **01.07.2015**
(21) Anmeldenummer: 07818754.9
(22) Anmeldetag: 05.10.2007
(51) Int. Cl.: C07C 233/18, C07C 231/02

(54) **VERFAHREN ZUR HERSTELLUNG VON FETTSÄUREALKANOLAMIDEN**
METHOD FOR PRODUCING FATTY ACID ALKANOL AMIDES
PROCÉDÉ DE PRODUCTION D'ALCANOLAMIDES D'ACIDES GRAS

(30) Priorität: 09.10.2006 DE 102006047615; 27.11.2006 DE 102006055856
(43) Veröffentlichungstag der Anmeldung: 22.07.2009
(73) Patentinhaber: Clariant Finance (BVI) Limited, Road Town, Tortola (VG)
(72) Erfinder: KRULL, Matthias, 55296 Harxheim (DE); MORSCHHÄUSER, Roman, 55122 Mainz (DE); KLUG, Peter, 63762 Grossostheim (DE); LERCH, Alexander, 63571 Gelnhausen (DE); KAYSER, Christoph, 55127 Mainz (DE); RITTER, Helmut, 42111 Wuppertal (DE); SCHMITZ, Sarah, 45130 Essen (DE)
(74) Vertreter: Mikulecky, Klaus
(86) Internationale Anmeldenummer: PCT/EP2007/008678
(87) Internationale Veröffentlichungsnummer: WO 2008/043493

(56) Entgegenhaltungen:
- US-A- 6 017 426
- US-A1- 2005 283 011
- MARTINEZ-PALOU, R. ET AL.: SYNLETT, Nr. 12, 2003, Seiten 1847-1849, XP002466232
- PLANTIER-ROYON, R.: C.R.CHIMIE, 2004, Seiten 119-123, XP002466233
- MASSICOT, F. ET AL.: SYNTHESIS, Nr. 16, 2001, Seiten 2441-2444, XP002466234 in der Anmeldung erwähnt
- KUAN JU LIU ET AL: "Lipase-Catalyzed Synthesis of Fatty Acid Diethanolamides", JOURNAL OF AGRICULTURAL AND FOOD CHEMISTRY, vol. 49, no. 12, 1 December 2001 (2001-12-01), pages 5761-5764, XP55065619, ISSN: 0021-8561, DOI: 10.1021/jf0107858

## Beschreibung

Fettsäurederivate, die funktionelle Gruppen mit hydrophilem Charakter tragen, finden weit verbreitete Verwendung als oberflächenaktive Substanzen. Eine wichtige Klasse derartiger oberflächenaktiver Substanzen sind nichtionische Amphiphile, die in großem Ausmaß als Emulgatoren, Korrosionsschutzmittel, Kühlschmiermittel in der Metallbearbeitung, als Lubricity-Additive in der Mineralölindustrie wie auch als Rohstoffe für die Herstellung von Waschmitteln, Reinigungskonzentraten, Detergentien, Kosmetika und Pharmazeutika eingesetzt werden.

Von besonderem Interesse sind dabei insbesondere solche Fettsäurederivate, die mindestens einen über eine Amidgruppe verknüpften Alkylrest tragen, der seinerseits mit mindestens einer hydrophilen Charakter verleihenden Hydroxylgruppe substituiert ist. Diese kann vor der eigentlichen Verwendung auch weiter derivatisiert werden, beispielsweise durch Umsetzung mit Alkylenoxiden wie Ethylen-, Propylen- oder Butylenoxid bzw. durch Oxidation mit geeigneten Oxidationsmitteln. Derartige Amide weisen eine gegenüber entsprechenden Estern stark erhöhte Hydrolysestabilität auf.

Um den wachsenden Bedarf für bestehende und neue Anwendungen zu decken, wurden verschiedene Methoden für die Herstellung Hydroxylgruppen tragender Fettsäureamide entwickelt. Bei der Herstellung derartiger Amide ist man dabei bisher auf kostenintensive und/oder langwierige Herstellverfahren angewiesen, um eine kommerziell interessante Ausbeute zu erzielen. Die gängigen Herstellverfahren erfordern aktivierte Carbonsäurederivate wie beispielsweise Säureanhydride, Säurehalogenide wie Säurechloride oder Ester, die mit Hydroxylgruppen tragenden Aminen, hier im Folgenden als Alkanolamine bezeichnet, umgesetzt werden bzw. eine in-situ-Aktivierung der Reaktanden durch den Einsatz von Kupplungsreagentien wie beispielsweise N,N'-Dicyclohexylcarbodiimid. Bei diesen Herstellverfahren entstehen zum Teil große Mengen unerwünschter Nebenprodukte wie Alkohole, Säuren und Salze, die vom Produkt abgetrennt und entsorgt werden müssen. Aber auch die in den Produkten verbleibenden Reste dieser Hilfs- und Nebenprodukte können zum Teil sehr unerwünschte Effekte bewirken. So führen beispielsweise Halogenidionen wie auch Säuren zu Korrosion; Kupplungsreagentien sowie die von ihnen gebildeten Nebenprodukte sind zum Teil giftig, sensibilisierend oder auch carcinogen.

Die direkte thermische Kondensation von Carbonsäure und Alkanolamin führt zu keinen befriedigenden Resultaten, da verschiedene Nebenreaktionen die Ausbeute mindern und teilweise auch die Produkteigenschaften beeinträchtigen. Problematisch ist dabei zum einen die Bisfunktionalität der Alkanolamine, die neben der Amidbildung in erheblichem Ausmaß zu Esterbildung Anlass gibt. Da Alkanolaminester andere Eigenschaften wie beispielsweise eine deutlich geringere Hydrolysestabilität aufweisen, sind sie als Nebenprodukt in den meisten Anwendungen unerwünscht. Darüber hinaus führen Esteramide, bei denen sowohl Amino- wie auch Hydroxylgruppe acyliert sind, in Tensidlösungen zu unerwünschten Trübungen. Der Esteranteil kann zwar zumindest teilweise durch thermisches Behandeln in Amide umgewandelt werden, wobei jedoch auf Grund der dazu erforderlichen langen Reaktionszeiten sehr oft Farbe und Geruch der so hergestellten Alkanolamide beeinträchtigt werden. Eine Abtrennung der Esteranteile wie auch der Esteramidanteile ist aber auf Grund der meist sehr ähnlichen physikalischen Eigenschaften nur schwer oder gar nicht möglich. Als weitere unerwünschte Nebenreaktionen werden beispielsweise eine Decarboxylierung der Carbonsäure und Oxidations- wie auch Eliminierungsreaktionen der Aminogruppe während des zur Erzielung hoher Umsätze erforderlichen langen Erhitzens beobachtet. In der Regel führen diese Nebenreaktionen zu gefärbten Nebenprodukten und es ist nicht möglich, insbesondere für kosmetische Anwendungen gewünschte farblose Produkte mit Hazen-Farbzahlen (gemäß DIN/ISO 6271) von beispielsweise weniger als 250 herzustellen. Letzteres erfordert zusätzliche Verfahrensschritte wie beispielsweise das Bleichen, das aber seinerseits den Zusatz weiterer Hilfsstoffe erfordert und oftmals zu einer ebenso unerwünschten Beeinträchtigung des Geruchs der Amide oder zu unerwünschten Nebenprodukten wie Peroxiden und deren Abbauprodukten führt.

Ein neuerer Ansatz zu Synthese von Amiden ist die durch Mikrowellen unterstützte Umsetzung von Carbonsäuren mit Aminen zu Amiden. So offenbaren Gelens et al., Tetrahedron Letters 2005, 46(21), 3751-3754, eine Vielzahl an Amiden, die unter Zuhilfenahme von Mikrowellenstrahlung synthetisiert wurden. Darunter findet sich auch Benzoesäuremonoethanolamid, das mit einer Ausbeute von 66 % erhalten wird.

Massicot et al., Synthesis 2001 (16), 2441-2444 beschreiben die Synthese von Diamiden der Weinsäure mit Ethanolamin, wobei eine 68 %ige Ausbeute an Diamid erzielt wird.

EP-A-0 884 305 offenbart die Amidierung von 2-Amino-octadecandiol-1,3 mit 2-Hydroxystearinsäure unter Mikrowellenbestrahlung, wobei Ceramide mit einer Ausbeute von ca. 70 % entstehen.

US-6017426 offenbart Umsetzungsprodukte von Fettsäuren mit Aminen der Formel: 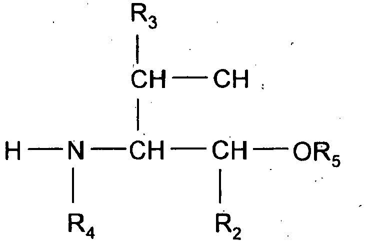 indem ein Aminoalkohol mit einer Carbonsäure unter Mikrowellenbestrahlung umgesetzt wird.

Martinez-Palou, R. et al.: Synlett, Nr. 12, 2003, Seiten 1847 - 1849 beschreibt die Synthese von langkettigen 2-Alkyl-1-(2-hydroxyethyl)-2-imidazolinen und deren Präkursoren (Amide) durch Reaktion zwischen Aminoethylethanolamin und Fettsäure in Gegenwart von CaO unter Mikrowellenbestrahlung.

Plantier-Royon, R.: C.R. Chimie, 2004, Seiten 119 - 123, XP002466233 lehrt ein Verfahren zur Herstellung von Diamiden der Weinsäure mittels Mikrowellenbestrahlung.

Eine weitere Umsatzerhöhung bei der Amidierung von Benzoesäure bzw. Hydroxycarbonsäuren und Alkanolaminen erscheint über den Weg der Mikrowellenbestrahlung auf Grund der als Nebenreaktion auftretenden Eliminierungen (Decarboxylierung von Benzoesäure; Wasserabspaltung aus Hydroxycarbonsäuren) nicht möglich. Diese Nebenreaktionen sind dabei unter kommerziellen wie auch ökologischen Aspekten besonders nachteilig, da die dabei gebildeten Nebenprodukte nicht in den Prozess recyclisiert werden können sondern aufwendig abgetrennt und entsorgt werden müssen.

Aufgabe der vorliegenden Erfindung war es, ein Verfahren zur Herstellung von Fettsäurealkanolamiden zu finden, bei dem Fettsäuren und Hydroxylgruppen tragende Amine direkt und in hohen, das heißt bis zu quantitativen Ausbeuten zu Fettsäurealkanolamiden umgesetzt werden können. Weiterhin sollen dabei keine bzw. nur untergeordnete Mengen an Nebenprodukten wie insbesondere Paraffine und/oder Olefine anfallen. Weiterhin sollen dabei Fettsäurealkanolamide mit möglichst geringer Eigenfärbung entstehen.

Überraschenderweise wurde gefunden, dass sich Fettsäurealkanolamide durch Mikrowellenbestrahlung von Ammoniumsalzen, die sich von Aminen, die mindestens eine primäre oder sekundäre Aminogruppe und mindestens eine Hydroxylgruppe tragen, und Fettsäuren ableiten, in hohen Ausbeuten herstellen lassen. Überraschenderweise treten dabei praktisch keine Decarboxilierung der Fettsäure und keine nennenswerte Esterbildung auf. Weiterhin zeigen die Fettsäureamide praktisch keine Eigenfärbung.

Gegenstand der Erfindung ist ein Verfahren zur Herstellung von Fettsäurealkanolamiden, indem mindestens ein Amin, das mindestens eine sekundäre Aminogruppe und mindestens eine Hydroxylgruppe enthalt, wobei das Amin der Formel

HNR¹R²

entspricht, worin
- R¹: für eine Gruppe der Formel -(B-O)ₘ-H
- B: für einen Alkylenrest mit 2 bis 10 C-Atomen,
- m: für eine Zahl von 1 bis 500 steht und
- R²: für R¹ oder einen Kohlenwasserstoffrest mit 1 bis 50 C-Atomen, welcher Heteroatome sowie Substituenten enthalten kann,
stehen,
mit mindestens einer Fettsäure zu einem Ammoniumsalz umgesetzt wird, und dieses Ammoniumsalz nachfolgend unter Mikrowellenbestrahlung weiter zum Alkanolamid umgesetzt wird.

Unter Alkanolamiden werden Amide verstanden, die sich von Fettsäuren ableiten und deren Amidstickstoffatom mindestens einen mit mindestens einer Hydroxylgruppe substituierten Kohlenwasserstoffrest trägt. Von Halogenidionen freie Fettsäureamide enthalten keine über die ubiquitären Mengen an Halogenidionen hinausgehenden Mengen dieser Ionen.

Der Begriff der Fettsäure wird in hier im Sinne von aliphatischer Monocarbonsäure verwendet. Unter Fettsäuren werden vorzugsweise Carbonsäuren verstanden, die einen Kohlenwasserstoffrest mit 1 bis 50 C-Atomen tragen. Bevorzugte Fettsäuren haben 4 bis 50, insbesondere 6 bis 30 und speziell 8 bis 24 C-Atome wie beispielsweise 12 bis 18 C-Atome. Sie können natürlichen oder synthetischen Ursprungs sein. Sie können Substituenten wie beispielsweise Halogenatome, halogenierte Alkylreste, Cyano-, Hydroxyalkyl-, Methoxy-, Nitril-, Nitro-, und/oder Sulfonsäuregruppen tragen mit der Maßgabe, dass diese unter den Reaktionsbedingungen stabil sind und keine Nebenreaktionen wie beispielsweise Eliminierungsreaktionen eingehen. Bevorzugt bestehen die Kohlenwasserstoffreste nur aus Kohlenstoff und Wasserstoff. Besonders bevorzugte aliphatische Kohlenwasserstoffreste können linear, verzweigt oder zyklisch sowie gesättigt oder ungesättigt sein. Sind sie ungesättigt, so können sie eine oder mehrere wie beispielsweise zwei, drei oder mehr Doppelbindungen enthalten. Bevorzugt befindet sich keine Doppelbindung in α,β-Position zur Carboxylgruppe. So hat sich das erfindungsgemäße Verfahren besonders zur Herstellung von Alkanolamiden mehrfach ungesättigter Fettsäuren bewährt, da die Doppelbindungen der ungesättigten Fettsäuren nicht angegriffen werden. Geeignete Fettsäuren sind beispielsweise Octan-, Decan-, Dodecan-, Tridecan-, Tetradecan-, 12-Methyltridecan-, Pentadecan-, 13-Methyltetradecan-, 12-Methyltetradecan-, Hexadecan-, 14-Methylpentadecan-, Heptadecan-, 15-Methylhexadecan-, 14-Methylhexadecan-, Octadecan-, Iso-Octadecan-, Icosan-, Docosan- und Tetracosansäure sowie Myristolein-, Palmitolein-, Hexadecadien-, Delta-9-cis-Heptadecen-, Öl-, Petroselin-, Vaccen-, Linol-, Linolen-, Gadolein-, Gondo-, Icosadien-, Arachidon-, Cetolein-, Eruca-, Docosadien- und Tetracosensäure sowie Ricinolsäure. Weiterhin geeignet sind aus natürlichen Fetten und Ölen wie beispielsweise Baumwollsamen-, Cocos-, Erdnuss-, Färberdistel-, Mais-, Palmkern-, Raps-, Ricinus-, Oliven-, Senfsamen, Soja-, Sonnenblumenöl sowie Talg-, Knochen- und Fischöl gewonnene Fettsäuremischungen. Als Fettsäuren bzw. Fettsäuremischungen für das erfindungsgemäße Verfahren ebenfalls geeignet sind Tallölfettsäure sowie Harz- und Naphthensäuren.

Erfindungsgemäß geeignete Alkanolamine besitzen mindestens eine sekundäre Aminogruppe, das heißt mindestens eine Aminogruppe trägt zwei Wasserstoffatome. Weiterhin trägt mindestens ein Alkylrest mindestens eine Hydroxylgruppe. Bevorzugte Amine entsprechen der Formel

HNR¹R²

worin
- R¹: für eine Gruppe der Formel

-(B-O)ₘ-H

worin
B für einen Alkylenrest mit 2 bis 10 C-Atomen und
m für eine Zahl von 1 bis 500 steht.
- R²: für R¹ oder einen Kohlenwasserstoffrest mit 1 bis 50 C-Atomen stehen.

Für den Fall, dass auch R² für R¹ steht, sind Amine bevorzugt, die insgesamt höchstens 5 und insbesondere 1, 2 oder 3 Hydroxylgruppen tragen.

Bevorzugt steht B für einen linearen oder verzweigten Alkylenrest mit 2 bis 5 C-Atomen, besonders bevorzugt für einen linearen oder verzweigten Alkylenrest mit 2 oder 3 C-Atomen und insbesondere für eine Gruppe der Formel -CH₂-CH₂-und/oder -CH(CH₃)-CH₂-.

Bevorzugt steht m für eine Zahl zwischen 2 und 300 und insbesondere für eine Zahl zwischen 3 und 100. In einer besonders bevorzugten Ausführungsform steht m für 1 oder 2. Bei Alkoxyketten mit m ≥ 3 und insbesondere mit m ≥ 5 kann es sich um eine Blockpolymerkette handeln, die alternierende Blöcke verschiedener Alkoxyeinheiten, vorzugsweise Ethoxy- und Propoxyeinheiten aufweist. Es kann sich dabei auch um eine Kette mit statistischer Abfolge der Alkoxyeinheiten öder um ein Homopolymer handeln.

In einer bevorzugten Ausführungsform steht R² für C₁-C₃₀-Alkyl, C₂-C₃₀-Alkenyl, C₅-C₁₂-Cycloalkyl, C₆-C₁₂-Aryl, C₇-C₃₀-Aralkyl oder eine heteroaromatische Gruppe mit 5 bis 12 Ringgliedern. Die Kohlenwasserstoffreste können Heteroatome wie beispielsweise Sauerstoff und Stickstoff enthalten sowie gegebenenfalls Substituenten wie beispielsweise Halogenatome, halogenierte Alkylreste, Nitro-, Cyano-, Nitril-, und/oder Aminogruppen tragen. Bevorzugt steht R² für Alkylreste mit 1 bis 18 C-Atomen, insbesondere mit 1 bis 8 C-Atomen und Alkenylreste mit 2 bis 18 C-Atomen, insbesondere mit 2 bis 8 C-Atomen. Diese Alkyl- und Alkenylreste können linear oder verzweigt sein. Geeignete Alkyl- und Alkenylreste sind beispielsweise Methyl, Ethyl, Propyl, iso-Propyl, n-Butyl, iso-Butyl, Hexy, Decyl, Dodecyl, Tetradecyl, Hexadecyl, Octadecyl, iso-Stearyl und Oleyl.

In einer weiteren bevorzugten Ausführungsform steht R² für einen Alkylrest mit 1 bis 4 C-Atomen wie beispielsweise Methyl oder Ethyl.

Besonders geeignet ist das erfindungsgemäße Verfahren zur Herstellung von Fettsäuredi(alkanol)amiden, in denen R² für eine Gruppe der Formel -(B-O)ₘ-H steht, in der die Bedeutungen für B und m in R¹ und R² gleich oder verschieden seien können. Insbesondere sind die Bedeutungen für R¹ und R² dabei gleich.

Beispiele für geeignete Alkanolamine sind 3-Amino-1-propanol, N-Methylaminoethanol, N-Ethylaminoethanol, N-Butylethanolamin, N-Methylisopropanolamin, 2-(2-Aminoethoxy)ethanol, 2-Amino-2-methyl-1-propanol, 3-Amino-2,2-dimethyl-1-propanol, 2-Amino-2-hydroxymethyl-1,3-propandiol, Diethanolamin, Dipropanolamin, Diisopropanolamin, Di(diethylenglykol)amin, N-(2-Aminoethyl)ethanolamin sowie Poly(ether)amine wie Poly(ethylenglykol)amin und Poly(propylenglykol)amin mit jeweils 4 bis 50 Alkylenoxideinheiten.

Das Verfahren ist insbesondere geeignet zur Herstellung von Laurinsäuremonoethanolamid, Laurinsäurediethanolamid, Laurinsäurediglykolamid, Cocosfettsäuremonoethanolamid, Cocosfettsäurediethanolamid, Cocosfettsäurediglykolamid, Stearinsäuremonoethanolamid, Stearinsäurediethanolamid, Stearinsäurediglykolamid, Tallölfettsäuremonoethanolamid, Tallölfettsäurediethanolamid und Tallölfettsäurediglykolamid.

Die erfindungsgemäß hergestellten Alkanolamide enthalten bezogen auf die Gesamtheit der vorhandenen Fettsäuren und Fettsäurederivate bevorzugt weniger als 5 mol-%, speziell weniger als 2 mol-% und insbesondere praktisch keine aus der Acylierung der Hydroxylgruppe des Alkanolamins resultierenden Ester bzw. Esteramide. Unter praktisch keine Ester und Alkanolaminester enthaltend werden Alkanolamide verstanden, deren Ester- und Esteramidgehalt unter 1 mol-% liegt und mit üblichen Analysenverfahren wie beispielsweise der ¹H-NMR-Spektroskopie nicht nachgewiesen werden kann.

Die Hazen-Farbzahl der erfindungsgemäß hergestellten Alkanolamide liegt bezogen auf 100 % Aktivsubstanz bevorzugt unterhalb 150 und insbesondere unterhalb 100.

Der Gesamtgehalt der erfindungsgemäß hergestellten Alkanolamide an Estern und Esteramiden liegt bevorzugt unter 2 mol-% wie beispielsweise unter 1 mol-%.

Im erfindungsgemäßen Verfahren können Fettsäure und Amin in beliebigen Verhältnissen miteinander zur Reaktion gebracht werden. Bevorzugt erfolgt die Umsetzung mit molaren Verhältnissen zwischen Fettsäure und Amin von 10:1 bis 1:10, bevorzugt von 2:1 bis 1:2, speziell von 1:1,2 bis 1,2:1 und insbesondere equimolar.

In vielen Fällen hat es sich als vorteilhaft erwiesen, mit einem geringen Überschuss an Amin, das heißt molaren Verhältnissen von Amin zu Fettsäure von mindestens 1,01 : 1,00 und insbesondere zwischen 1,02 : 1,00 und 1,3 : 1,0 wie beispielsweise zwischen 1,05 : 1,0 und 1,1 : 1 zu arbeiten. Dabei wird die Fettsäure praktisch quantitativ zum Alkanolamid umgesetzt. Besonders vorteilhaft ist dieses Verfahren, wenn das eingesetzte, mindestens eine primäre und/oder sekundäre und mindestens eine Hydroxylgruppe tragende Alkanolamin leicht flüchtig ist. Leicht flüchtig heißt hier, dass das Amin einen Siedepunkt bei Normaldruck von vorzugsweise unterhalb 200°C wie beispielsweise unterhalb 150°C besitzt und sich somit destillativ vom Amid abtrennen lässt.

Die erfindungsgemäße Herstellung der Amide erfolgt durch Umsetzung der Fettsäure und des Alkanolamins zum Ammoniumsalz und nachfolgender Bestrahlung des Salzes mit Mikrowellen. Das Ammoniumsalz wird dabei bevorzugt in-situ erzeugt und nicht isoliert. Bevorzugt wird der durch die Mikrowellenbestrahlung bedingte Temperaturanstieg durch Regelung der Mikrowellenintensität und/oder Kühlung des Reaktionsgefäßes auf maximal 300°C begrenzt. Besonders bewährt hat sich die Durchführung der Umsetzung bei Temperaturen zwischen 100 und maximal 250°C und speziell zwischen 120 und maximal 200°C wie beispielsweise bei Temperaturen zwischen 125 und 180°C.

Die Dauer der Mikrowellenbestrahlung hängt von verschiedenen Faktoren wie dem Reaktionsvolumen, der Geometrie des Reaktionsraumes und dem gewünschten Umsetzungsgrad ab. Üblicherweise wird die Mikrowellenbestrahlung über einen Zeitraum von weniger als 30 Minuten, bevorzugt zwischen 0,01 Sekunden und 15 Minuten, besonders bevorzugt zwischen 0,1 Sekunden und 10 Minuten und insbesondere zwischen einer Sekunde und 5 Minuten wie beispielsweise zwischen 5 Sekunden und 2 Minuten vorgenommen. Die Intensität (Leistung) der Mikrowellenstrahlung wird dabei so eingestellt, dass das Reaktionsgut in möglichst kurzer Zeit die angestrebte Reaktionstemperatur erreicht. Zum anschließenden Aufrechterhalten der Temperatur kann das Reaktionsgut mit reduzierter und/oder gepulster Leistung weiter bestrahlt werden. Zur Einhaltung der Maximaltemperatur bei gleichzeitig größtmöglicher Mikrowelleneinstrahlung hat es sich bewährt, das Reaktionsgut beispielsweise mittels Kühlmantel, im Reaktionsraum befindliche Kühlrohre, durch intermittierende Kühlung zwischen verschiedenen Bestrahlungszonen und/oder durch Siedekühlung über externe Wärmetauscher zu kühlen. In einer bevorzugten Ausführungsform wird das Umsetzungsprodukt direkt nach Beendigung der Mikrowellenbestrahlung möglichst schnell auf Temperaturen unterhalb 120°C, bevorzugt unterhalb 100°C und speziell unterhalb 60°C abgekühlt.

Bevorzugt wird die Umsetzung bei Drücken zwischen 10⁴ und 2·10⁷ Pa und speziell zwischen 10⁵ Pa (Atmosphärendruck) und 5·10⁶ Pa durchgeführt. Besonders bewährt hat sich das Arbeiten in geschlossenen Gefäßen, in denen oberhalb des Siedepunkts der Edukte bzw. Produkte, des gegebenenfalls anwesenden Lösemittels und/oder oberhalb des während der Reaktion gebildeten Reaktionswassers gearbeitet wird. Üblicherweise reicht der sich auf Grund der Erwärmung des Reaktionsansatzes einstellende Druck zur erfolgreichen Durchführung des erfindungsgemäßen Verfahrens aus. Es kann aber auch unter erhöhtem Druck und/oder unter Anlegen eines Druckprofils gearbeitet werden. In einer weiteren bevorzugten Variante des erfindungsgemäßen Verfahrens wird unter Atmosphärendruck, wie er sich zum Beispiel im offenen Gefäß einstellt, gearbeitet.

Zur Vermeidung von Nebenreaktionen und zur Herstellung von möglichst reinen Produkten hat es sich bewährt, das erfindungsgemäße Verfahren in Gegenwart eines inerten Schutzgases wie beispielsweise Stickstoff, Argon oder Helium durchzuführen.

In einer bevorzugten Ausführungsform wird zur Beschleunigung bzw. zur Vervollständigung der Reaktion in Gegenwart von dehydratisierenden Katalysatoren gearbeitet. Vorzugsweise arbeitet man dabei in Gegenwart eines sauren anorganischen, metallorganischen oder organischen Katalysators oder Gemischen aus mehreren dieser Katalysatoren.

Als saure anorganische Katalysatoren im Sinne der vorliegenden Erfindung sind beispielsweise Schwefelsäure, Phosphorsäure, Phosphonsäure, hypophosphorige Säure, Aluminiumsulfathydrat, Alaun, saures Kieselgel und saures Aluminiumhydroxid zu nennen. Weiterhin sind beispielsweise Aluminiumverbindungen der allgemeinen Formel Al(OR⁵)₃ und Titanate der allgemeinen Formel Ti(OR⁵)₄ als saure anorganische Katalysatoren einsetzbar, wobei die Reste R⁵ jeweils gleich oder verschieden sein können und unabhängig voneinander gewählt sind aus C₁-C₁₀-Alkylresten, beispielsweise Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, iso-Amyl, n-Hexyl, sec.-Hexyl, n-Heptyl, n-Octyl, 2-Ethylhexy, n-Nonyl oder n-Decyl, C₃-C₁₂-Cycloalkylresten, beispielsweise Cyclopropyl, Cyclobutyl, Cyclopentyl, Cyclohexyl, Cycloheptyl, Cyclooctyl, Cyclononyl, Cyclodecyl, Cycloundecyl und Cyclododecyl; bevorzugt sind Cyclopentyl, Cyclohexyl und Cycloheptyl. Bevorzugt sind die Reste R⁵ in Al(OR⁵)₃ bzw. Ti(OR⁵)₄ jeweils gleich und gewählt aus Isopropyl, Butyl und 2-Ethylhexyl.

Bevorzugte saure metallorganische Katalysatoren sind beispielsweise gewählt aus Dialkylzinnoxiden (R⁵)₂SnO, wobei R⁵ wie oben stehend definiert ist. Ein besonders bevorzugter Vertreter für saure metallorganische Katalysatoren ist Di-n-butylzinnoxid, das als so genanntes Oxo-Zinn oder als Fascat^{®}-Marken kommerziell erhältlich ist.

Bevorzugte saure organische Katalysatoren sind saure organische Verbindungen mit beispielsweise Phosphatgruppen, Sulfonsäuregruppen, Sulfatgruppen oder Phosphonsäuregruppen. Besonders bevorzugte Sulfonsäuren enthalten mindestens eine Sulfonsäuregruppe und mindestens einen gesättigten oder ungesättigten, linearen, verzweigten und/oder zyklischen Kohlenwasserstoffrest mit 1 bis 40 C-Atomen und bevorzugt mit 3 bis 24 C-Atomen. Insbesondere bevorzugt sind aromatische Sulfonsäuren, speziell alkylaromatische Mono-Sulfonsäuren mit einem oder mehreren C₁-C₂₈-Alkylresten und insbesondere solche mit C₃-C₂₂-Alkylresten. Geeignete Beispiele sind Methansulfonsäure, Butansulfonsäure, Benzolsulfonsäure, p-Toluolsulfonsäure, Xylolsulfonsäure, 2-Mesitylensulfonsäure, 4-Ethylbenzolsulfonsäure, Isopropylbenzolsulfonsäure, 4-Butylbenzolsulfonsäure, 4-Octylbenzolsulfonsäure; Dodecylbenzolsulfonsäure, Didodecylbenzolsulfonsäure, Naphthalinsulfonsäure. Auch saure Ionenaustauscher können als saure organische Katalysatoren eingesetzt werden, beispielsweise Sulfonsäuregruppenhaltige Poly(styrol)-Harze, die mit etwa 2 mol-% Divinylbenzol vernetzt sind.

Besonders bevorzugt für die Durchführung des erfindungsgemäßen Verfahrens sind Borsäure, Phosphorsäure, Polyphosphorsäure und Polystyrolsulfonsäuren. Insbesondere bevorzugt sind Titanate der allgemeinen Formel Ti(OR⁵)₄ und speziell Titantetrabutylat und Titantetraisopropylat.

Wünscht man saure anorganische, metallorganische oder organische Katalysatoren einzusetzen, so setzt man erfindungsgemäß 0,01 bis 10 Gew.-%, bevorzugt 0,02 bis 2 Gew.-% Katalysator ein. In einer besonders bevorzugten Ausführungsform wird ohne Katalysator gearbeitet.

In einer weiteren bevorzugten Ausführungsform wird die Mikrowellenbestrahlung in Gegenwart von sauren, festen Katalysatoren durchgeführt. Dabei wird der feste Katalysator in dem gegebenenfalls mit Lösemittel versetzten Ammoniumsalz suspendiert oder bei kontinuierlichen Verfahren Vorteilhafterweise das gegebenenfalls mit Lösemittel versetzte Ammoniumsalz über einen Festbettkatalysator geleitet und Mikrowellenstrahlung ausgesetzt. Geeignete feste Katalysatoren sind beispielsweise Zeolithe, Kieselgel, Montmorillonit und (teil)vernetzte Polystyrolsulfonsäure, die gegebenenfalls mit katalytisch aktiven Metallsalzen imprägniert sein können. Geeignete saure Ionentauscher auf Basis von Polystyrolsulfonsäuren, die als Festphasenkatalysatoren eingesetzt werden können, sind beispielsweise von der Firma Rohm&Haas unter der Markenbezeichnung Amberlyst^{®} erhältlich.

Es hat sich bewährt, in Gegenwart von Lösemitteln zu arbeiten um beispielsweise die Viskosität des Reaktionsmediums abzusenken, das Reaktionsgemisch, sofern es heterogen ist, zu fluidisieren und/oder die Wärmeabfuhr beispielsweise mittels Siedekühlung zu verbessern. Dafür können prinzipiell alle Lösemittel eingesetzt werden, die unter den angewendeten Reaktionsbedingungen inert sind und nicht mit den Edukten bzw. den gebildeten Produkten reagieren. Ein wichtiger Faktor bei der Auswahl geeigneter Lösemittel ist deren Polarität, die einerseits die Löseeigenschaften und andererseits das Ausmaß der Wechselwirkung mit Mikrowellenstrahlung bestimmt. Ein besonders wichtiger Faktor bei der Auswahl geeigneter Lösemittel ist deren dielektrischer Verlust ε". Der dielektrische Verlust ε" beschreibt den Anteil an Mikrowellenstrahlung, der bei der Wechselwirkung einer Substanz mit Mikrowellenstrahlung in Wärme überführt wird. Letzt genannter Wert hat sich als besonders wichtiges Kriterium für die Eignung eines Lösemittels für die Durchführung des erfindungsgemäßen Verfahrens erwiesen. Besonders bewährt hat sich das Arbeiten in Lösemitteln, die eine möglichst geringe Mikrowellenabsorption zeigen und somit nur einen kleinen Beitrag zur Erwärmung des Reaktionssystems liefern. Für das erfindungsgemäße Verfahren bevorzugte Lösemittel besitzen einen bei Raumtemperatur und 2450 MHz gemessenen dielektrischen Verlust ε" von weniger als 10 und vorzugsweise weniger als 1 wie beispielsweise weniger als 0,5. Eine Übersicht über den dielektrischen Verlust verschiedener Lösemittel findet sich zum Beispiel in "Microwave Synthesis" von B. L. Hayes, CEM Publishing 2002. Für das erfindungsgemäße Verfahren geeignet sind insbesondere Lösemittel mit ε"-Werten unterhalb 10 wie N-Methylpyrrolidon, N,N-Dimethylformamid oder Aceton, und insbesondere Lösemittel mit ε"-Werten unterhalb 1. Beispiele für besonders bevorzugte Lösemittel mit ε"-Werten unterhalb 1 sind aromatische und/oder aliphatische Kohlenwasserstoffe wie beispielsweise Toluol, Xylol, Ethylbenzol, Tetralin, Hexan, Cyclohexan, Decan, Pentadecan, Dekalin sowie kommerzielle Kohlenwasserstoffgemische wie Benzinfraktionen, Kerosin, Solvent Naphtha, ^{®}Shellsol AB, ^{®}Solvesso 150, ^{®}Solvesso 200, ^{®}Exxsol, ^{®}Isopar und ^{®}Shellsol-Typen. Lösemittelgemische, die ε"-Werte bevorzugt unterhalb 10 und speziell unterhalb 1 aufweisen, sind für die Durchführung des erfindungsgemäßen Verfahrens gleichermaßen bevorzugt. Prinzipiell ist das erfindungsgemäße Verfahren auch in Lösemitteln mit ε"-Werten von 10 und höher möglich, doch erfordert dies besondere Maßnahmen zur Einhaltung der Maximaltemperatur und führt oftmals zu verminderten Ausbeuten. Sofern in Gegenwart von Lösemitteln gearbeitet wird, liegt deren Anteil an der Reaktionsmischung bevorzugt zwischen 2 und 95 Gew.-%, speziell zwischen 5 und 90 Gew.-% und insbesondere zwischen 10 und 75 Gew.-% wie beispielsweise zwischen 30 und 60 Gew.-%. Besonders bevorzugt wird die Reaktion lösemittelfrei durchgeführt.

Die Mikrowellenbestrahlung wird üblicherweise in Geräten durchgeführt, die einen Reaktionsraum aus einem für Mikrowellen weitestgehend transparenten Material besitzen, in den in einem Mikrowellengenerator erzeugte Mikrowellenstrahlung über geeignete Antennensysteme eingekoppelt wird. Mikrowellengeneratoren, wie beispielsweise das Magnetron und das Klystron sind dem Fachmann bekannt.

Als Mikrowellen werden elektromagnetische Strahlen mit einer Wellenlänge zwischen etwa 1 cm und 1m und Frequenzen zwischen etwa 300 MHz und 30 GHz bezeichnet. Dieser Frequenzbereich ist prinzipiell für das erfindungsgemäße Verfahren geeignet. Bevorzugt wird für das erfindungsgemäße Verfahren Mikrowellenstrahlung mit den für industrielle, wissenschaftliche und medizinische Anwendungen freigegebenen Frequenzen von 915 MHz, 2,45 GHz, 5,8 GHz oder 27,12 GHz verwendet. Es kann sowohl im Mono- bzw. Quasi-Monomode wie auch im Multimode gearbeitet werden. Beim Monomode, der hohe Anforderungen an Geometrie und Größe von Apparatur und Reaktionsraum stellt, wird dabei durch eine stehende Welle insbesondere an deren Maximum eine sehr hohe Energiedichte erzeugt. Beim Multimode dagegen wird der gesamte Reaktionsraum weitgehend homogen bestrahlt, was zum Beispiel größere Reaktionsvolumina ermöglicht.

Die für die Durchführung des erfindungsgemäßen Verfahrens in das Reaktionsgefäß einzustrahlende Mikrowellenleistung ist insbesondere abhängig von der Geometrie des Reaktionsraums und damit des Reaktionsvolumens sowie der Dauer der erforderlichen Bestrahlung. Sie liegt üblicherweise zwischen 100 W und mehreren 100 kW und insbesondere zwischen 200 W und 100 kW wie beispielsweise zwischen 500 W und 70 kW. Sie kann an einer oder mehreren Stellen des Reaktors appliziert werden. Sie kann über einen oder mehrere Mikrowellengeneratoren erzeugt werden.

Die Reaktion kann diskontinuierlich im Batch-Verfahren oder, bevorzugt, kontinuierlich zum Beispiel in einem Strömungsrohr durchgeführt werden. Sie kann weiterhin in semi-Batch Prozessen wie beispielsweise kontinuierlich betriebenen Rührreaktoren oder Kaskadenreaktoren durchgeführt werden. In einer bevorzugten Ausführungsform wird die Reaktion in einem geschlossenen Gefäß durchgeführt, wobei das sich bildende Kondensat sowie gegebenenfalls Edukte und, sofern anwesend, Lösemittel zu einem Druckaufbau führen. Nach Beendigung der Reaktion kann der Überdruck durch Entspannung zur Verflüchtigung und Abtrennung von Reaktionswasser und gegebenenfalls Lösemittel sowie überschüssigen Edukten und/oder Abkühlung des Reaktionsprodukts verwendet werden. In einer weiteren Ausführungsform wird das gebildete Reaktionswasser nach dem Abkühlen und/oder Entspannen durch übliche Verfahren wie beispielsweise Phasentrennung, Destillation und/oder Absorption abgetrennt. Das erfindungsgemäße Verfahren kann ebenso erfolgreich in einem offenen Gefäß unter Siedekühlung und/oder Auskreisen des Reaktionswassers erfolgen.

In einer bevorzugten Ausführungsform wird das erfindungsgemäße Verfahren in einem diskontinuierlichen Mikrowellenreaktor durchgeführt. Dabei wird die Mikrowellenbestrahlung in einem gerührten Gefäß vorgenommen. Bevorzugt befinden sich zur Abführung überschüssiger Wärme im Reaktionsgefäß Kühlelemente wie beispielsweise Kühlfinger oder Kühlschlangen oder an das Reaktionsgefäß angeflanscht Rückflusskühler zur Siedekühlung des Reaktionsmediums. Für die Bestrahlung größerer Reaktionsvolumina wird die Mikrowelle hier bevorzugt im Multimode betrieben. Die diskontinuierliche Ausführungsform des erfindungsgemäßen Verfahrens erlaubt durch Variation der Mikrowellenleistung schnelle wie auch langsame Heizraten und insbesondere das Halten der Temperatur über längere Zeiträume wie beispielsweise mehrere Stunden. Die Reaktanden und gegebenenfalls Lösemittel und weitere Hilfsstoffe können vor Beginn der Mikrowellenbestrahlung im Reaktionsgefäß vorgelegt werden. Bevorzugt haben sie dabei Temperaturen unterhalb 100°C wie beispielsweise zwischen 10°C und 50°C. In einer bevorzugten Ausführungsform werden die Reaktanden oder Teile davon dem Reaktionsgefäß erst während der Bestrahlung mit Mikrowellen zugeführt. In einer weiteren bevorzugten Ausführungsform wird der diskontinuierliche Mikrowellenreaktor unter kontinuierlichem Zuführen von Edukten und gleichzeitigem Ausschleusen von Reaktionsgut in Form eines Semi-Batch- bzw. Kaskadenreaktors betrieben.

In einer besonders bevorzugten Ausführungsform wird das erfindungsgemäße Verfahren in einem kontinuierlichen Mikrowellenreaktor durchgeführt. Die Reaktionsmischung wird dazu durch ein druckfestes, gegenüber den Reaktanden inertes, für Mikrowellen weitestgehend transparentes und in einen Mikrowellenofen eingebautes Reaktionsrohr geführt. Dieses Reaktionsrohr hat bevorzugt einen Durchmesser von einem Millimeter bis ca. 50 cm, speziell zwischen 1 mm und 35 cm wie beispielsweise zwischen 2 mm und 15 cm. Unter Reaktionsrohren werden hier Gefäße verstanden, deren Verhältnis von Länge zu Durchmesser größer als 5, bevorzugt zwischen 10 und 100.000, besonders bevorzugt zwischen 20 und 10.000 wie beispielsweise zwischen 30 und 1.000 ist. In einer speziellen Ausführungsform ist das Reaktionsrohr in Form eines Doppelmantelrohres ausgestaltet durch dessen Innen- und Außenraum, die Reaktionsmischung nacheinander im Gegenstrom geführt werden kann, um beispielsweise die Temperaturführung und Energieeffizienz des Verfahrens zu erhöhen. Als Länge des Reaktionsrohres ist dabei die vom Reaktionsgemisch insgesamt durchströmte Strecke zu verstehen. Das Reaktionsrohr ist auf seiner Länge von mindestens einem, bevorzugt aber mehreren wie beispielsweise zwei, drei, vier, fünf, sechs, sieben acht oder mehr Mikrowellenstrahlern umgeben. Die Mikrowelleneinstrahlung erfolgt bevorzugt über den Rohrmantel. In einer weiteren bevorzugten Ausführungsform erfolgt die Mikrowelleneinstrahlung mittels mindestens einer Antenne über die Rohrenden. Das Reaktionsrohr ist üblicherweise am Einlass mit einer Dosierpumpe sowie einem Manometer und am Auslass mit einem Druckhalteventil und einem Wärmetauscher versehen. Bevorzugt werden die Edukte Alkanolamin und Fettsäure, beide unabhängig voneinander gegebenenfalls mit Lösemittel verdünnt, erst kurz vor dem Eintritt in das Reaktionsrohr vermischt. Weiterhin bevorzugt werden die Edukte dem erfindungsgemäßen Verfahren in flüssiger Form mit Temperaturen unterhalb 100°C wie beispielsweise zwischen 10°C und 50°C zugeführt. Dazu können höher schmelzende Edukte beispielsweise in geschmolzenem Zustand oder mit Lösemittel versetzt eingesetzt werden.

Durch Variation von Rohrquerschnitt, Länge der Bestrahlungszone (hierunter wird der Anteil des Reaktionsrohres verstanden, in dem das Reaktionsgut Mikrowellenstrahlung ausgesetzt ist), Fließgeschwindigkeit, Geometrie der Mikrowellenstrahler, der eingestrahlten Mikrowellenleistung sowie der dabei erreichten Temperatur werden die Reaktionsbedingungen so eingestellt, dass die maximale Reaktionstemperatur schnellstmöglich erreicht wird und die Verweilzeit bei Maximaltemperatur so kurz bleibt, dass so wenig Neben- oder Folgereaktionen wie möglich auftreten. Bevorzugt wird der kontinuierliche Mikrowellenreaktor im Monomode oder Quasi-Monomode betrieben. Die Verweilzeit im Reaktionsrohr liegt dabei im Allgemeinen unter 30 Minuten, bevorzugt zwischen 0,01 Sekunden und 15 Minuten und bevorzugt zwischen 0,1 Sekunden und 5 Minuten, wie beispielsweise zwischen einer Sekunde und 3 Minuten. Das Reaktionsgut kann zur Vervollständigung der Reaktion, gegebenenfalls nach Zwischenkühlung, mehrfach den Reaktor durchlaufen. Es hat sich besonders bewährt, wenn das Reaktionsprodukt unmittelbar nach Verlassen des Reaktionsrohres z. B. durch Mantelkühlung oder Entspannung abgekühlt wird.

Dabei war es besonders überraschend, dass trotz der im kontinuierlich durchflossenen Strömungsrohr nur sehr kurzen Verweildauer des Ammoniumsalzes im Mikrowellenfeld eine so weitgehende Amidierung ohne Bildung nennenswerter Mengen an Nebenprodukten stattfindet. Bei einer entsprechenden Umsetzung dieser Ammoniumsalze in einem Strömungsrohr unter thermischer Mantelheizung werden nur geringe Umsätze zum Amid erzielt, wobei jedoch gleichzeitig signifikante Mengen an Estern sowie Esteramiden gebildet werden. Zudem führen die zur Erzielung geeigneter Reaktionstemperaturen benötigten extrem hohen Wandtemperaturen zu Zersetzungsreaktionen und der Bildung gefärbter Spezies.

Zur Vervollständigung der Umsetzung hat es sich in vielen Fällen bewährt, das erhaltene Rohprodukt nach Entfernen von Reaktionswasser sowie gegebenenfalls Austragen von Produkt und/oder Nebenprodukt erneut der Mikrowellenbestrahlung auszusetzen.

Üblicherweise fallen über den erfindungsgemäßen Weg hergestellte Alkanolamide in einer für die weitere Verwendung ausreichenden Reinheit an. Für spezielle Anforderungen können sie jedoch nach üblichen Reinigungsverfahren wie Destillation, Umkristallisation, Filtration bzw. chromatographische Verfahren weiter aufgereinigt werden.

Die erfindungsgemäß hergestellten basischen Amide eignen sich beispielsweise als Emulgatoren, in der Erdölindustrie als Korrosions- oder Gashydratinhibitoren und als Schmierverbesserer in Schmier- und Brennstoffölen sowie in der Metallbearbeitung als Kühlschmiermittel. Eventuell vorhandene endständige Hydroxylgruppen können bei Bedarf anschließend weiter derivatisiert werden, beispielsweise durch Veresterung, Veretherung und andere bekannte Reaktionen.

Das erfindungsgemäße Verfahren erlaubt eine sehr schnelle und kostengünstige Herstellung von Fettsäurealkanolamiden in hohen Ausbeuten und mit hoher Reinheit. Insbesondere weisen sie nur einen geringen Gehalt an Alkanolaminestern wie auch an Esteramiden auf. Ihre wässrigen Lösungen sind daher klar und weisen im Gegensatz zu entsprechenden, durch thermische Kondensation hergestellte Fettsäurealkanolamide keine durch Esteramide verursachten Trübungen auf. Die Eigenfärbung der erfindungsgemäß hergestellten Amide entspricht Hazen-Farbzahlen (nach DIN/ISO 6271) von unter 200 und teilweise unter 150 wie beispielsweise unterhalb 100, wogegen nach klassischen Methoden Hazen-Farbzahlen unterhalb 250 ohne zusätzliche Verfahrensschritte nicht zugänglich sind. Darüber hinaus fallen keine wesentlichen Mengen an Nebenprodukten wie beispielsweise decarboxylierte Carbonsäuren an. Derartig schnelle und selektive Umsetzungen sind nach klassischen Methoden nicht zu erzielen und waren alleine durch Heizen auf hohe Temperaturen auch nicht zu erwarten. Da die nach dem erfindungsgemäßen Verfahren hergestellten Alkanolamide zudem verfahrensbedingt keine Reste von Kupplungsreagentien bzw. deren Folgeprodukten enthalten, können sie problemlos auch in toxikologisch sensiblen Bereichen wie beispielsweise kosmetischen und pharmazeutischen Zubereitungen eingesetzt werden.

### Beispiele

Die Umsetzungen unter Mikrowellenbestrahlung erfolgten in einem Single-Mode Mikrowellenreaktor vom Typ "Discover" der Firma CEM bei einer Frequenz von 2,45 GHz. Die Kühlung der Reaktionsgefäße erfolgte mittels Druckluft. Die Temperaturmessung erfolgte über einen IR-Sensor am Küvettenboden. Die Temperaturmessungen mussten auf Grund der Druckbedingungen in den Reaktionsgefäßen über einen IR-Sensor am Küvettenboden erfolgen. Durch Vergleichsversuche mit einer in die Reaktionsmischung eintauchenden Glasfiberoptik wurde festgestellt, dass die Temperatur im Reaktionsmedium im hier relevanten Temperaturbereich etwa 50 bis 80°C über der mit dem IR-Sensor am Küvettenboden gemessenen Temperatur liegt.

Die diskontinuierlich durchgeführten Umsetzungen erfolgten in geschlossenen, druckfesten Glasküvetten mit einem Volumen von 8 ml unter Magnetrührung. Kontinuierlich durchgeführte Umsetzungen erfolgten in druckfesten, zylindrischen, als Doppelmantelrohr ausgestalteten Glasküvetten (ca. 10 x 1,5 cm; Reaktionsvolumen 15 ml) mit innen liegendem, über dem Küvettenboden endendem Einleitungsrohr (Bodeneinlass) und Produktabnahme am oberen Ende der Küvette. Der sich während der Reaktion aufbauende Druck wurde über ein Druckhalteventil auf maximal 20 bar begrenzt und in eine Vorlage entspannt. Das Ammoniumsalz wurde durch das Einleitungsrohr in die Küvette gepumpt und die Verweilzeit in der Bestrahlungszone durch Modifizierung der Pumpenleistung eingestellt.

Die Analytik der Produkte erfolgte mittels ¹H-NMR-Spektroskopie bei 500 MHz ein CDCl₃. Wasserbestimmungen erfolgten mittels Karl-Fischer-Titration. Hazen-Farbzahlen wurden nach DIN/ISO 6271 bestimmt.

### Beispiel 1: Herstellung von Cocosfettsäuremonoethanolamid (Vergleich)

Unter Kühlen und Rühren wurde 1,5 g Ethanolamin langsam mit 5,0 g geschmolzener Cocosfettsäure versetzt und gemischt. Nach Abklingen der Wärmetönung wurde das so erhaltene Ammoniumsalz in einer geschlossenen Küvette für 10 Minuten unter maximaler Kühlleistung einer Mikrowellenbestrahlung von 200 W ausgesetzt. Es wurde eine mittels IR-Sensor gemessene Temperatur von 195°C erreicht, wobei der Druck auf 10 bar anstieg.

Das erhaltene Rohprodukt enthielt als Hauptkomponenten 85 % Cocosfettsäuremonoethanolamid, 5,4 % Wasser sowie unumgesetzte Edukte. Nach Trocknung des Reaktionsansatzes über MgSO₄, erneute fünfminütige Bestrahlung mit Mikrowellen von 200 W und Trocknung über MgSO₄ wurde eine Ausbeute an Cocosfettsäuremonoethanolamid von über 98 % erhalten. Das so erhaltene Cocosfettsäuremonoethanolamid enthielt weniger als 1 mol-% Aminoester und Esteramid. Die Hazen-Farbzahl lag bei 80 (unverdünntes geschmolzenes Produkt).

### Beispiel 2: Herstellung von Laurinsäurediethanolamid

Bei 50°C wurden 2,5 g Diethanolamin unter Rühren langsam mit 4,6 g geschmolzener Laurinsäure versetzt und gemischt. Nach Abklingen der Wärmetönung wurde das so erhaltene Ammoniumsalz in einer geschlossenen Küvette für 10 Minuten unter maximaler Kühlleistung einer Mikrowellenbestrahlung von 200 W ausgesetzt. Es wurde eine mittels IR-Sensor gemessene Temperatur von 190°C erreicht, wobei der Druck auf 10 bar stieg.

Das Rohprodukt enthielt als Hauptkomponente 78 % Laurinsäurediethanolamid, 4,5 % Wasser und unumgesetzte Edukte. Nach Trocknung des Reaktionsansatzes über MgSO₄, erneute fünfminütige Bestrahlung mit Mikrowellen von 200 W, destillativer Entfernung von Reaktionswasser und überschüssigem Diethanolamin unter Vakuum wurde eine Ausbeute an Laurinsäurdiethanolamid von über 97 % erhalten. Das erhaltene Cocosfettsäurediethanolamid enthielt etwa 1 mol-% Aminoester und Esteramid. Die Hazen-Farbzahl lag bei 120 (unverdünntes, geschmolzenes Produkt).

### Beispiel 3: Herstellung von N-Lauroyl-2-(2-aminoethoxy)ethanol (Vergleich)

Unter Kühlen und Rühren wurden 2,5 g 2-(2-Aminoethoxy)ethanol langsam mit einer equimolaren Menge an Laurinsäure versetzt und gemischt. Nach Abklingen der Wärmetönung wurde das so erhaltene Ammoniumsalz in einer geschlossenen Küvette für 5 Minuten unter maximaler Kühlleistung einer Mikrowellenbestrahlung von 150 W ausgesetzt. Es wurde eine mittels IR-Sensor gemessene Temperatur von 200°C erreicht wobei der Druck auf 12 bar stieg.

Das Rohprodukt enthielt als Hauptkomponente 80 % N-Lauroyl-2-(2-aminoethoxy)ethanol, 4,7 % Wasser sowie unumgesetzte Edukte. Nach Trocknung des Reaktionsansatzes über MgSO₄, erneute zweiminütige Bestrahlung mit Mikrowellen von 150 W und Trocknung wurde von N-Lauroyl-2-(2-aminoethoxy)ethanol mit über 97 % Ausbeute erhalten. Das erhaltene N-Lauroyl-2-(2-aminoethoxy)ethanol enthielt weniger als 1 mol-% Aminoester und Esteramid. Die Hazen-Farbzahl lag bei 90 (unverdünntes Produkt).

### Beispiel 4: Kontinuierliche Herstellung von Cocosfettsäurediethanolamid

Unter Kühlung und Rühren wurden 105 g Diethanolamin bei 40°C langsam mit 205 g Cocosfettsäure versetzt. Nach Abklingen der Wärmetönung wurde das so erhaltene Ammoniumsalz kontinuierlich über den Bodeneinlass durch die in der Mikrowellencavität montierte, druckfeste Glasküvette gepumpt. Die Förderleistung der Pumpe wurde dabei so eingestellt, dass die Verweilzeit in der Küvette und damit in der Bestrahlungszone etwa 2 Minuten betrug. Es wurde unter maximaler Kühlleistung mit einer Mikrowellenleistung von 200 W gearbeitet, wobei eine mittels IR-Sensor gemessene Temperatur von 180°C erreicht wurde. Nach Verlassen der Glasküvette wurde die Reaktionsmischung über einen kurzen Liebig-Kühler auf 40°C abgekühlt.

Nach Abtrennen des Reaktionswassers wurde das Rohprodukt nochmals wie oben durch die Glasküvette gepumpt und dabei erneut Mikrowellenstrahlung ausgesetzt. Nach Abtrennen des Reaktionswassers wurde Cocosfettsäurediethanolamid mit über 97 % Ausbeute erhalten. Es konnten keine Esteranteile nachgewiesen werden. Das erhaltene Cocosfettsäurediethanolamid enthielt weniger als 1 mol-% Aminoester und Esteramid. Die Hazen-Farbzahl dieses Amids lag bei 90 (unverdünntes Produkt).

### Beispiel 5: Kontinuierliche Herstellung von Cocosfettsäurepropanolamid (Vergleich)

Unter Kühlung und Rühren wurden 75 g Propanolamin (1 mol) bei 40°C langsam mit 214 g (1 mol) Cocosfettsäure versetzt. Nach Abklingen der Wärmetönung wurde das so erhaltene Ammoniumsalz kontinuierlich über den Bodeneinlass durch die in der Mikrowellencavität montierte, druckfeste Glasküvette gepumpt. Die Förderleistung der Pumpe wurde dabei so eingestellt, dass die Verweilzeit in der Küvette und damit in der Bestrahlungszone etwa 1,5 Minuten betrug. Es wurde unter maximaler Kühlleistung mit einer Mikrowellenleistung von 300 W gearbeitet, wobei eine mittels IR-Sensor gemessene Temperatur von 195°C erreicht wurde. Nach Verlassen der Glasküvette wurde die Reaktionsmischung über einen kurzen Liebig-Kühler auf etwa 100°C abgekühlt und danach das Reaktionswasser im Vakuum abgetrennt.

Nach Ersatz des unumgesetzten, ebenfalls abgetrennten Isopropanols wurde das Rohprodukt anschließend nochmals wie oben durch die Glasküvette gepumpt und dabei erneut Mikrowellenstrahlung ausgesetzt. Nach erneutem Abtrennen des Reaktionswassers wurde Cocosfettsäurepropanolamid mit über 95 % Ausbeute erhalten. Gemäß ¹H-NMR-Spektrum enthielt das Produkt noch etwa 3 % unreagierte Cocosfettsäure; die Konzentration eventueller weiterer Nebenprodukte (z.B. Ester, Esteramide) lagen unter der Nachweisgrenze. Die Hazen-Farbzahl dieses Amids lag bei 70 (unverdünntes, geschmolzenes Produkt).

### Beispiel 6: Kontinuierliche Herstellung von Tallölfettsäurediglykolamid (Vergleich)

Unter Kühlung und Rühren wurden 119 g Diglykolamin (1 mol) bei 50°C langsam mit 280 g (1 mol) Tallölfettsäure versetzt. Nach Abklingen der Wärmetönung wurde das so erhaltene Ammoniumsalz kontinuierlich über den Bodeneinlass durch die in der Mikrowellencavität montierte, druckfeste Glasküvette gepumpt. Die Förderleistung der Pumpe wurde dabei so eingestellt, dass die Verweilzeit in der Küvette und damit in der Bestrahlungszone etwa 2,5 Minuten betrug. Es wurde unter maximaler Kühlleistung mit einer Mikrowellenleistung von 200 W gearbeitet, wobei eine mittels IR-Sensor gemessene Temperatur von 190°C erreicht wurde. Nach Verlassen der Glasküvette wurde die Reaktionsmischung über einen kurzen Liebig-Kühler auf etwa 100°C abgekühlt und danach das Reaktionswasser im Vakuum abgetrennt. Das Rohprodukt wurde anschließend noch zweimal wie oben beschrieben durch die Glasküvette gepumpt und dabei erneut Mikrowellenstrahlung ausgesetzt und anschließend jeweils vom Reaktionswasser befreit.

Nach somit dreimaliger Bestrahlung der Reaktionsmischung mit Mikrowellen wurde ein Tallölfettsäurediglykolamid mit einer Reinheit von 96 % (¹H-NMR) erhalten. Gemäß ¹H-NMR-Spektrum enthielt das Produkt noch etwa 2 % unreagierte Tallölfettsäure; die Konzentrationen eventueller weiterer Nebenprodukte (z.B. Ester, Esteramide) lagen unter der Nachweisgrenze. Die Hazen-Farbzahl dieses Amids lag bei 80.

### Beispiel 7: Kontinuierliche thermische Umsetzung von Laurinsäure mit Ethanolamin (Vergleichsbeispiel)

Unter Kühlung und Rühren wurden 61 g Ethanolamin (1 mol) langsam mit 214 g Laurinsäure (1 mol) versetzt. Nach Abklingen der Wärmetönung wurde das so erhaltene Ammoniumsalz kontinuierlich über den Bodeneinlass durch eine sich in einem 250°C heißen Ölbad befindliche druckfeste Glasküvette gepumpt. Die Förderleistung der Pumpe wurde dabei so eingestellt, dass die Verweilzeit der Reaktanden in der Küvette und damit in der Reaktionszone etwa 90 Sekunden betrug. Eine Temperaturmessung wurde am Überlauf der Küvette vorgenommen. Die hier beobachteten Maximaltemperaturen 190°C. Nach Verlassen der Glasküvette wurde die Reaktionsmischung über einen kurzen Liebig-Kühler auf Raumtemperatur abgekühlt.

Mit Hilfe dieser kontinuierlichen Arbeitsweise unter Verwendung eines konventionellen Heizmediums konnten lediglich Umsätze von 10 % zum gewünschten Produkt erzielt werden. Daneben wurden 3 % des korrespondierenden Esters im ¹H-NMR nachgewiesen. Esteramide als Nebenprodukt konnten aufgrund des geringen Umsatzes nicht eindeutig identifiziert werden. Die Hazen-Farbzahl dieses Umsetzungsprodukts lag bei 280.

## Patentansprüche

1. Verfahren zur Herstellung von Fettsäurealkanolamiden, indem mindestens ein Amin, das mindestens eine sekundäre Aminogruppe und mindestens eine Hydroxylgruppe enthält, wobei das Amin der Formel
HNR¹R²
entspricht, worin
R¹ für eine Gruppe der Formel -(B-O)ₘ-H
B für einen Alkylenrest mit 2 bis 10 C-Atomen,
m für eine Zahl von 1 bis 500 steht und
R² für R¹ oder einen Kohlenwasserstoffrest mit 1 bis 50 C-Atomen, welcher Heteroatome sowie Substituenten enthalten kann,
stehen,
mit mindestens einer Fettsäure zu einem Ammoniumsalz umgesetzt wird, und dieses Ammoniumsalz nachfolgend unter Mikrowellenbestrahlung weiter zum Alkanolamid umgesetzt wird.

2. Verfahren nach Anspruch 1, worin die Fettsäure einen aliphatischen, Kohlenwasserstoffrest mit 1 bis 50 Kohlenstoffatomen umfasst.

3. Verfahren nach Anspruch 2, worin der Kohlenwasserstoffrest mindestens einen Substituenten ausgewählt aus Halogenatomen, halogenierten Alkylresten, Cyano-, Hydroxyalkyl-, Hydroxyl-, Methoxy-, Nitril-, Nitro- und Sulfonsäuregruppen umfasst.

4. Verfahren nach Anspruch 2 und/oder 3, worin der Kohlenwasserstoffrest gesättigt ist.

5. Verfahren nach Anspruch 2 und/oder 3, worin der Kohlenwasserstoffrest mindestens eine Doppelbindung umfasst.

6. Verfahren nach einem oder mehreren der Ansprüche 1 bis 5, worin die Fettsäure aus der Gruppe bestehend aus Octan-, Decan-, Dodecan-, Tridecan-, Tetradecan-, 12-Methyltridecan-, Pentadecan-, 13-Methyltetradecan-, 12-Methyltetradecan-, Hexadecan-, 14-Methylpentadecan-, Heptadecan-, 15-Methylhexadecan-, 14-Methylhexadecan-, Octadecan-, Iso-Octadecan-, Icosan-, Docosan- und Tetracosan-, Myristolein-, Palmitolein-, Hexadecadien-, Delta-9-cis-Heptadecen-, Öl-, Petroselin-, Vaccen-, Linol-, Linolen-, Gadolein-, Gondo-, Icosadien-, Arachidon-, Cetolein-, Eruca-, Docosadien-, Tetracosen-, Ricinol-, Tallölfett-, Harz- und Naphthensäuren ausgewählt ist.

7. Verfahren nach einem oder mehreren der Ansprüche 1 bis 6, worin das Amin aus der Gruppe bestehend aus N-Methylaminoethanol, N-Ethylaminoethanol, N-Butylethanolamin, N-Methylisopropanolämin, Diethanolamin, Dipropanolamin, Diisopropanolamin und Di(diethylenglykol)amin ausgewählt ist.

8. Verfahren nach einem oder mehreren der Ansprüche 1 bis 7, worin die Mikrowellenbestrahlung in Gegenwart eines dehydratisierenden Katalysators durchgeführt wird.

9. Verfahren nach einem oder mehreren der Ansprüche 1 bis 8, welches in Gegenwart eines Lösemittels durchgeführt wird.

10. Verfahren nach Anspruch 9, wobei das Lösemittel einen ε"-Wert von unter 10 aufweist.

11. Verfahren nach einem oder mehreren der Ansprüche 1 bis 10, welches bei Temperaturen unterhalb 300°C durchgeführt wird.

12. Verfahren nach einem oder mehreren der Ansprüche 1 bis 11, wobei die Reaktion bei Drücken zwischen 10⁴ und 2·10⁷ Pa durchgeführt wird.

13. Verfahren nach einem oder mehreren der Ansprüche 1 bis 12, wobei die Umsetzung kontinuierlich durch Bestrahlung mit Mikrowellen in einem vom Ammoniumsalz durchströmten Reaktionsrohr erfolgt.

14. Verfahren nach Anspruch 13, wobei das Reaktionsrohr aus einem nicht metallischen, mikrowellentransparenten Werkstoff besteht.

15. Verfahren nach Anspruch 13 und/oder 14, worin die Verweilzeit des Reaktionsgutes im Reaktionsrohr unter 30 Minuten beträgt.

16. Verfahren nach einem oder mehreren der Ansprüche 13 bis 15, wobei das Reaktionsrohr ein Verhältnis von Länge zu Durchmesser von mindestens 5 besitzt.

17. Verfahren nach einem oder mehreren der Ansprüche 1 bis 16, wobei B für einen linearen Alkylenrest mit 2 bis 5 C-Atomen steht.

## Claims

1. A process for preparing fatty acid alkanolamides by reacting at least one amine which contains at least one secondary amino group and at least one hydroxyl group, wherein the amine corresponds to the formula
HNR¹R²,
in which
R¹ is a group of the formula -(B-O)ₘ-H
B is an alkylene radical having 2 to 10 carbon atoms,
m is from 1 to 500 and
R² is R¹ or a hydrocarbon radical having 1 to 50 carbon atoms, which may contain heteroatoms and substituents,
with at least one fatty acid to give an ammonium salt, and this ammonium salt is subsequently converted further under microwave irradiation to the alkanolamide.

2. The process as claimed in claim 1, in which the fatty acid comprises an aliphatic hydrocarbon radical having 1 to 50 carbon atoms.

3. The process as claimed in claim 2, in which the hydrocarbon radical comprises at least one substituent selected from halogen atoms, halogenated alkyl radicals, or cyano, hydroxyalkyl, hydroxyl, methoxy, nitrile, nitro and sulfonic acid groups.

4. The process as claimed in claim 2 and/or 3, in which the hydrocarbon radical is saturated.

5. The process as claimed in claim 2 and/or 3, in which the hydrocarbon radical comprises at least one double bond.

6. The process as claimed in one or more of claims 1 to 5, in which the fatty acid is selected from the group consisting of octanoic acid, decanoic acid, dodecanoic acid, tridecanoic acid, tetradecanoic acid, 12-methyltridecanoic acid, pentadecanoic acid, 13-methyltetradecanoic acid, 12-methyltetradecanoic acid, hexadecanoic acid, 14-methylpentadecanoic acid, heptadecanoic acid, 15-methylhexadecanoic acid, 14-methylhexadecanoic acid, octadecanoic acid, isooctadecanoic acid, eicosanoic acid, docosanoic acid and tetracosanoic acid, myristoleic acid, palmitoleic acid, hexadecadienoic acid, delta-9-cis-heptadecenoic acid, oleic acid, petroselic acid, vaccenic acid, linoleic acid, linolenic acid, gadoleic acid, gondoleic acid, eicosadienoic acid, arachidonic acid, cetoleic acid, erucic acid, docosadienoic acid, tetracosenoic acid, ricinoleic acid, tall oil fatty acid, resin acids and naphthenic acids.

7. The process as claimed in one or more of claims 1 to 6, in which the amine is selected from the group consisting of N-methylaminoethanol, N-ethylaminoethanol, N-butylethanolamine, N-methylisopropanolamine, diethanolamine, dipropanolamine, diisopropanolamine and di(diethylene glycol)amine.

8. The process as claimed in one or more of claims 1 to 7, wherein the microwave irradiation is performed in the presence of a dehydrating catalyst.

9. The process as claimed in one or more of claims 1 to 8, which is performed in the presence of a solvent.

10. The process as claimed in claim 9, wherein the solvent has an ε" value of less than 10.

11. The process as claimed in one or more of claims 1 to 10, which is performed at temperatures below 300°C.

12. The process as claimed in one or more of claims 1 to 11, wherein the reaction is performed at pressures between 10⁴ and 2.10⁷ Pa.

13. The process as claimed in one or more of claims 1 to 12, wherein the reaction is effected continuously by irradiating with microwaves in a reaction tube through which the ammonium salt flows.

14. The process as claimed in claim 13, wherein the reaction tube consists of a nonmetallic microwave-transparent material.

15. The process as claimed in claim 13 and/or 14, in which the residence time of the reaction mixture in the reaction tube is less than 30 minutes.

16. The process as claimed in one or more of claims 13 to 15, wherein the reaction tube has a ratio of length to diameter of at least 5.

17. The process as claimed in one or more of claims 1 to 16, wherein B is a linear alkylene radical having 2 to 5 carbon atoms.

## Revendications

1. Procédé de fabrication d'alcanolamides d'acides gras, selon lequel au moins une amine, qui contient au moins un groupe amino secondaire et au moins un groupe hydroxyle, l'amine correspondant à la formule
HNR¹R²
dans laquelle
R¹ représente un groupe de formule -(B-O)ₘ-H,
B représente un radical alkylène de 2 à 10 atomes C,
m représente un nombre de 1 à 500, et
R² représente R¹ ou un radical hydrocarboné de 1 à 50 atomes C, qui peut contenir des hétéroatomes et des substituants,
est mise en réaction avec au moins un acide gras pour former un sel d'ammonium, puis ce sel d'ammonium est transformé en l'alcanolamide sous exposition à des micro-ondes.

2. Procédé selon la revendication 1, dans lequel l'acide gras comprend un radical hydrocarboné aliphatique de 1 à 50 atomes de carbone.

3. Procédé selon la revendication 2, dans lequel le radical hydrocarboné comprend au moins un substituant choisi parmi les atomes d'halogène, les radicaux alkyle halogénés, les groupes cyano, hydroxyalkyle, hydroxyle, méthoxy, nitrile, nitro et acide sulfonique.

4. Procédé selon la revendication 2 et/ou 3, dans lequel le radical hydrocarboné est saturé.

5. Procédé selon la revendication 2 et/ou 3, dans lequel le radical hydrocarboné comprend au moins une double liaison.

6. Procédé selon une ou plusieurs des revendications 1 à 5, dans lequel l'acide gras est choisi dans le groupe constitué par l'acide octanoïque, décanoïque, dodécanoïque, tridécanoïque, tétradécanoïque, 12-méthyltridécanoïque, pentadécanoïque, 13-méthyltétradécanoïque, 12-méthyltétradécanoïque, hexadécanoïque, 14-méthylpentadécanoïque, heptadécanoïque, 15-méthylhexadécanoïque, 14-méthylhexadécanoïque, octadécanoïque, iso-octadécanoïque, icosanoïque, docosanoïque et tétracosanoïque, myristoléique, palmitoléique, hexadécadiénique, delta-9-cis-heptadécénoïque, oléique, pétrosélinique, vaccénique, linoléique, linolénique, gadoléique, gondoïque, icosadiénique, arachidonique, cétoléique, érucique, docosadiénique, tétracosénique, ricinoléique, gras de tallöl, résinique et naphténique.

7. Procédé selon une ou plusieurs des revendications 1 à 6, dans lequel l'amine est choisie dans le groupe constitué par le N-méthylaminoéthanol, le N-éthylaminoéthanol, la N-butyléthanolamine, la N-méthylisopropanolamine, la diéthanolamine, la dipropanolamine, la diisopropanolamine et la di(diéthylène glycol)amine.

8. Procédé selon une ou plusieurs des revendications 1 à 7, dans lequel l'exposition à des micro-ondes est réalisée en présence d'un catalyseur déshydratant.

9. Procédé selon une ou plusieurs des revendications 1 à 8, qui est réalisé en présence d'un solvant.

10. Procédé selon la revendication 9, dans lequel le solvant présente une valeur ε'' inférieure à 10.

11. Procédé selon une ou plusieurs des revendications 1 à 10, qui est réalisé à des températures inférieures à 300 °C.

12. Procédé selon une ou plusieurs des revendications 1 à 11, dans lequel la réaction est réalisée à des pressions comprises entre 10⁴ et 2·10⁷ Pa.

13. Procédé selon une ou plusieurs des revendications 1 à 12, dans lequel la réaction a lieu en continu par exposition à des micro-ondes dans un tube de réaction traversé par le sel d'ammonium.

14. Procédé selon la revendication 13, dans lequel le tube de réaction est constitué d'un matériau non métallique, transparent aux micro-ondes.

15. Procédé selon la revendication 13 et/ou 14, dans lequel le temps de séjour du mélange réactionnel dans le tube de réaction est inférieur à 30 minutes.

16. Procédé selon une ou plusieurs des revendications 13 à 15, dans lequel le tube de réaction présente un rapport entre la longueur et le diamètre d'au moins 5.

17. Procédé selon une ou plusieurs des revendications 1 à 16, dans lequel B représente un radical alkylène linéaire de 2 à 5 atomes C.
